# FASCICULE DE BREVET EUROPEEN

(11) **EP 3 376 982 B1**
(45) Date de publication et mention de la délivrance du brevet: **16.10.2019**
(21) Numéro de dépôt: 16806265.1
(22) Date de dépôt: 08.11.2016
(51) Int. Cl.: A61B 17/86

(54) **VIS OSSEUSE A DOUBLE FILET**
ZWEIGÄNGIGE KNOCHENSCHRAUBE
DOUBLE-THREADED BONE SCREW

(30) Priorité: 16.11.2015 FR 1560934
(43) Date de publication de la demande: 26.09.2018
(73) Titulaire: Clariance, 62217 Beaurains (FR)
(72) Inventeur: KRIER, Brice, 62000 Dainville (FR); VIRGAUX, Nicolas, 75018 Paris (FR); SAUVAGE, Bruno, 62690 Frevin Capelle (FR); VIART, Guy, 62128 Saint Leger (FR); ROKEGEM, Pascal, 62000 Arras (FR); CLEVENNA, Andrew, Dallas, Texas 75225 (US)
(74) Mandataire: Godard, Xavier
(86) Numéro de dépôt international: PCT/FR2016/052896
(87) Numéro de publication internationale: WO 2017/085376

(56) Documents cités:
- DE-B3-102014 208 012
- US-A1- 2014 012 334
- US-A1- 2014 200 620
- US-B2- 9 179 955

## Description

La présente invention est relative à une vis osseuse à double filet qui est destinée à être vissée dans de l'os pour assurer, par exemple, la fixation de prothèses ou d'implant.
On connait d'après l'enseignement décrit dans les brevets US 3 703 843 et US 3 861 269 une vis de fixation permettant la fixation de pièces l'une contre l'autre qui sont constituées de matériaux différents présentant des résistances différentes.

La vis de fixation comporte une tête d'entraînement à partir de laquelle se prolonge un corps cylindrique pourvue de deux filets cylindriques de pas identique. Le corps cylindrique comporte sur 1/3 de sa longueur deux filets cylindriques disposés soit au niveau de son extrémité libre pointue (US 3 703 843), soit juste en dessous de la tête d'entraînement (US 3 861 269).
On connait également d'après l'enseignement décrit dans le brevet EP 1991145 une vis à os comprenant un corps fileté à profil cylindro-conique pourvu à l'une de ses extrémités d'une tête d'entraînement ou d'un connecteur pour la fixation d'une tige de liaison d'un dispositif d'ostéosynthèse.
Le corps cylindro-conique comporte en dessous de sa tête d'entraînement ou du connecteur une première section filetée comprenant deux filets hélicoïdaux de même pas se prolongeant par une seconde section filetée constituée d'un seul filet hélicoïdale issu de la première section filetée.

Le brevet US 9179955 décrit une vis à os dont le corps fileté cylindro-conique comporte des parties d'ancrage de profil différents.

Dans le cas de vis à os, on note que le tissu osseux dans lequel est implantée ladite vis présente des résistances différentes entre sa partie centrale constituée d'os spongieux et sa partie périphérique formée d'os cortical.
En effet, le tissu osseux est constitué d'une composante dure et dense, l'os cortical qui enveloppe une composante trabéculaire, l'os spongieux, moins dense et moins dur. Ces deux composantes forment un continuum présentant un gradient de densité qui augmente en se rapprochant de l'os cortical.
Pour cela, la vis osseuse à double filet discontinu suivant la présente invention a pour objet d'améliorer la tenue osseuse et comprend le long de son corps d'ancrage des parties filetées discontinues assurant sur toute la longueur dudit corps d'ancrage la fixation de ladite vis dans les différentes composantes du tissu osseux.

La bonne tenue mécanique de la vis osseuse suivant la présente invention dans le tissu osseux est assurée d'une part par la prise du filet dans l'os cortical et l'os spongieux et d'autre part par une encapsulation d'os cortical et d'os spongieux autour de la vis et plus particulièrement entre les pas du filet pour venir épouser parfaitement le profil de ladite vis.
La vis osseuse à double filet discontinu pour la fixation d'une prothèse ou d'un implant contre une partie osseuse suivant la présente invention comporte une tête d'entraînement se prolongeant par un corps d'ancrage constitué d'une âme cylindro-conique sur laquelle s'étendent au moins deux filets cylindro-coniques assurant l'ancrage osseux de ladite vis, le corps d'ancrage comprenant sur toute sa longueur et à partir de la tête d'entraînement des parties d'ancrage de profil différents assurant un ancrage correspondant aux différentes duretés et résistances de l'os, ledit corps d'ancrage comprenant en dessous de la tête d'entraînement d'entraînement une première partie d'ancrage constituée d'une âme conique et deux filets à profil cylindrique de pas identique, une seconde partie d'ancrage formée d'une âme cylindrique et d'un seul filet cylindrique sur ¼ de tour, une troisième partie d'ancrage pourvue d'une âme cylindrique et de deux filets à profil cylindriques sur ½ tour, une quatrième partie d'ancrage comprenant une âme cylindrique et un d'un seul filet à profil cylindrique sur **n** tours en fonction de la longueur totale de la vis osseuse, et une cinquième partie d'ancrage comportant une âme conique et deux filets à profil conique formant la pointe de la vis osseuse.
La vis osseuse à double filet discontinu suivant la présente invention comporte une première partie d'ancrage comprenant deux filets à profil cylindrique de pas identique décalés sur la périphérie de l'âme conique de 180° degré.
La vis osseuse à double filet discontinu suivant la présente invention comporte une quatrième partie d'ancrage comprenant un filet cylindrique qui est différent du filet cylindrique prévu pour la seconde partie.
La vis osseuse à double filet discontinu suivant la présente invention comporte un alésage interne.

La vis osseuse à double filet discontinu suivant la présente invention comporte une tête d'entraînement qui est constituée d'une tête à profil sphérique présentant dans sa partie interne une empreinte pour la mise en place d'un outil.
La vis osseuse à double filet discontinu suivant la présente invention comporte une tête d'entraînement qui est constituée d'une tête monobloc présentant un profil en forme de U destiné à recevoir une vis serrage pour l'immobilisation.
La vis osseuse à double filet discontinu suivant la présente invention comporte une cinquième partie d'ancrage à profil conique comprenant des filets auto taraudants.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figure 1 est une vue en perspective illustrant la vis osseuse à double filet discontinu suivant la présente invention.
Figures 2 et 3 sont des vues de face et en coupe représentant la vis osseuse à double filet suivant la présente invention.
Figures 4 et 5 sont des vues de face et en coupe montrant une variante de réalisation de la tête d'entraînement de vis osseuse à double filet discontinu suivant la présente invention.

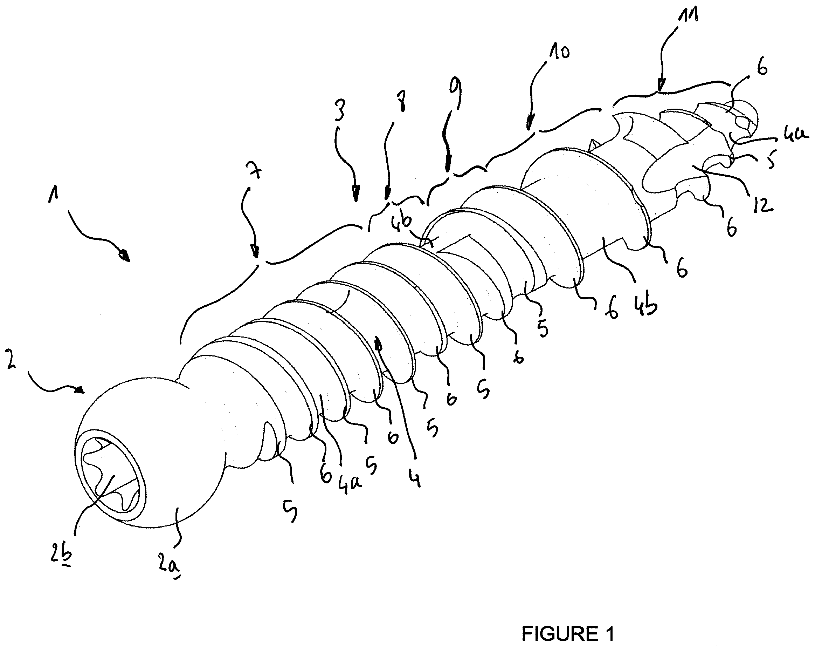

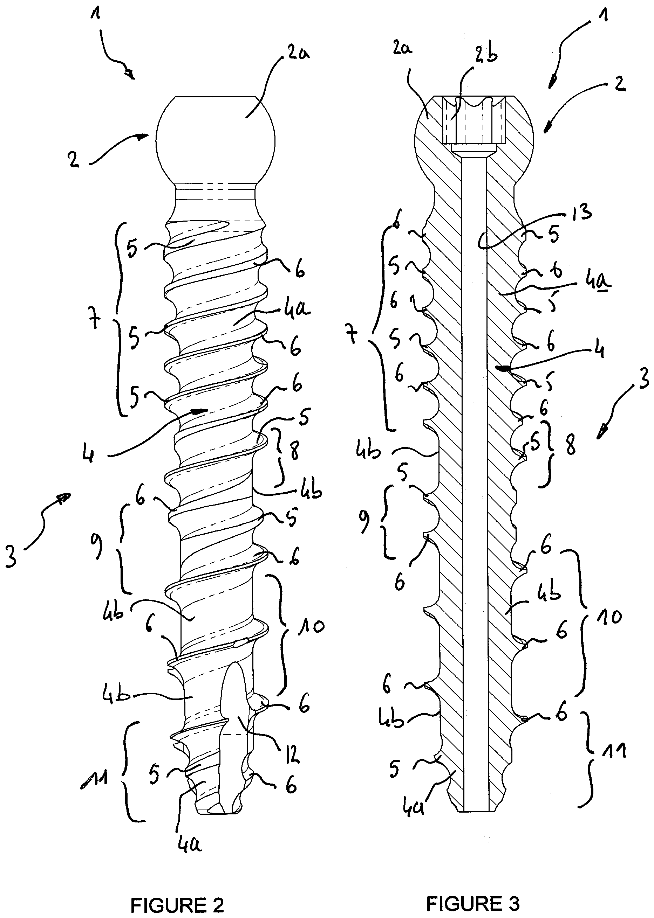

On a montré en figures 1 à 3 une vis osseuse 1 à double filet discontinu 5, 6 permettant d'assurer, par exemple, la fixation d'une prothèse ou d'un implant contre une partie osseuse.

La vis osseuse 1 comporte une tête d'entraînement 2 se prolongeant par un corps d'ancrage 3 constitué d'une âme cylindro-conique 4 sur laquelle s'étendent au moins deux filets cylindro-coniques discontinus 5, 6 assurant l'ancrage de ladite vis 1 dans les différentes composantes du tissu osseux.

Dans cette première illustration, la tête d'entraînement 2 peut être constituée d'une tête à profil sphérique 2**a** présentant dans sa partie interne une empreinte 2**b** pour la mise en place d'un outil assurant l'entraînement en rotation de la vis osseuse 1.
Le corps d'ancrage 3 comprend sur toute sa longueur et à partir de la tête d'entraînement 2 des parties d'ancrage 7, 8, 9, 10 présentant respectivement des profils différents de manière à assurer un ancrage correspondant aux différentes duretés et résistances du tissu osseux

A cet effet, le corps d'ancrage 3 comporte en dessous de la tête d'entraînement 2 une première partie d'ancrage 7 qui est constituée de l'âme 4 dont le profil à ce niveau est conique 4**a** et de deux filets 5, 6 à profil cylindriques et de pas identique.
Les deux filets 5, 6 à profil cylindrique sont décalés sur la périphérie de l'âme conique 4**a** de 180° degrés. Dans cette première partie d'ancrage 7, la vis osseuse 1 présente un corps d'ancrage 3 à double filet permettant d'assurer une prise et un maintien de cette dernière dans l'os cortical du tissu osseux.

Le corps d'ancrage 3 comprend dans le prolongement de la première partie d'ancrage 7 une seconde partie d'ancrage 8 formée de l'âme 4 dont le profil dans cette zone est cylindrique 4**b** et d'un seul filet cylindrique 5 positionné sur ¼ de tour dudit corps.

Dans cette seconde partie d'ancrage 8 le second filet cylindrique 6 est arrêté pour ne laisser que le premier filet cylindrique 5 venir en contact avec le tissu osseux lors de la mise en place de la vis 1.

Le corps d'ancrage 3 comporte dans le prolongement de la seconde partie d'ancrage 8 une troisième partie d'ancrage 9 pourvue de l'âme 4 dont le profil cylindrique 4**b** est prolongé et de deux filets 5, 6 à profil cylindriques aménagés sur ½ tour dudit corps.

Dans cette troisième partie d'ancrage 9 le second filet cylindrique 6 est repris suite à son arrêt dans la partie d'ancrage précédente, tandis que le premier filet cylindrique 5 est maintenu de manière continue depuis la première partie d'ancrage 7.

Ainsi, la vis osseuse 1 présente au niveau de cette troisième partie d'ancrage 9 un corps d'ancrage 3 à double filet sur ½ tour afin d'augmenter son empreinte dans le tissu osseux.

Le corps d'ancrage 3 comprend dans le prolongement de la troisième partie d'ancrage 9 une quatrième partie d'ancrage 10 formée de l'âme 4 dont le profil est cylindrique 4**b** depuis la seconde partie d'ancrage 8 et d'un seul filet 6 à profil cylindrique qui s'étend sur **n** tour en fonction de la longueur totale de la vis osseuse 1.

Dans cette quatrième partie d'ancrage 10 le premier filet cylindrique 5 est arrêté pour ne laisser que le second filet cylindrique 6 venir en contact avec le tissu osseux lors de la mise en place de la vis 1. On note que la quatrième partie d'ancrage 10 comprend un filet cylindrique 6 qui est différent du filet cylindrique 5 prévu pour la seconde partie 8.

Le corps d'ancrage 3 comporte dans le prolongement de la quatrième partie d'ancrage 10 une cinquième et dernière partie d'ancrage 11 constituée de l'âme 4 dont le profil est à nouveau conique 4**a** et des deux filets 5, 6 dont le profil est également conique afin de former avec le profil de l'âme 4 la pointe de la vis osseuse 1.

Dans cette cinquième partie d'ancrage 11, la vis osseuse 1 présente un corps d'ancrage 3 à double filet permettant d'assurer la pénétration de cette dernière dans l'os cortical du tissu osseux.

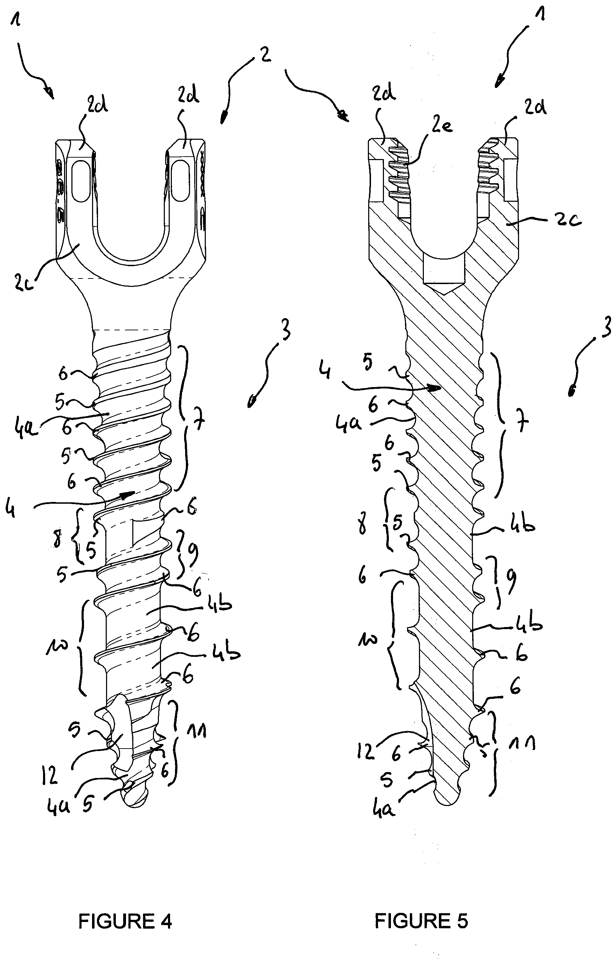

Egalement, la cinquième partie d'ancrage 11 de la vis osseuse 1 et plus particulièrement sa pointe comporte des empreintes 12 coupant verticalement le profil des deux filets 5 et 6.
Chaque empreinte 12 positionnée sur la périphérie de la pointe de la vis osseuse 1 permet de constituer sur les filets 5 et 6 des bords d'attaques tranchants 5**a** et 6**a** disposés dans le sens de rotation et d'introduction de ladite vis pour rendre lesdits filets auto-taraudants.
Selon une première variante la vis osseuse 1 à double filet discontinu 5, 6 peut comporter un alésage interne 13 permettant son guidage dans les tissus osseux au moyen d'un guide non représenté et préalablement introduit dans ledit tissus osseux.
En figures 4 et 5, on a illustré une vis osseuse 1 comportant une tête d'entraînement 2 se prolongeant par un corps d'ancrage 3 constitué d'une âme cylindro-conique 4 sur laquelle s'étendent au moins deux filets cylindro-coniques discontinus 5, 6 assurant l'ancrage de ladite vis 1 dans les différentes composantes du tissu osseux.

Dans cette variante, la tête d'entraînement 2 est constituée d'une tête monobloc 2**c** présentant un profil en forme de U solidaire du corps d'ancrage 3 présentant les parties d'ancrage 7, 8, 9, 10 de profils différents de manière à assurer un ancrage correspondant aux différentes duretés et résistance du tissu osseux.

La tête monobloc 2**c** présentant un profil en forme de U dont la partie interne des branches verticales 2**d** comporte un filetage 2**e** coopérant avec une vis serrage non représentée pour l'immobilisation, par exemple, d'une tige de liaison d'un dispositif d'ostéosynthèse rachidien.

Il doit d'ailleurs être entendu que la description qui précède n'a été donnée qu'à titre d'exemple, et qu'elle ne limite nullement le domaine de l'invention qui est défini par les revendications.

## Revendications

1. Vis osseuse (1) à double filet discontinu pour la fixation d'une prothèse ou d'un implant contre une partie osseuse, comportant une tête d'entraînement d'entraînement (2) se prolongeant par un corps d'ancrage (3) constitué d'une âme cylindro-conique (4) sur laquelle s'étendent au moins deux filets cylindro-coniques (5, 6) assurant l'ancrage osseux de ladite vis (1), le corps d'ancrage (3) comprenant sur toute sa longueur et à partir de la tête d'entraînement (2) des parties d'ancrage de profil différents assurant un ancrage correspondant aux différentes duretés et résistances de l'os, **caractérisé en ce que** ledit corps d'ancrage (3) comporte en dessous de la tête d'entraînement (2) une première partie d'ancrage (7) constituée d'une âme conique (4**a**) et deux filets (5, 6) à profil cylindrique de pas identique, une seconde partie d'ancrage (8) formée d'une âme cylindrique (4**b**) et d'un seul filet cylindrique (5) sur ¼ de tour, une troisième partie d'ancrage (9) pourvue d'une âme cylindrique (4**b**) et de deux filets (5, 6) à profil cylindriques sur ½ tour, une quatrième partie d'ancrage (10) comprenant une âme cylindrique (4**b**) et un seul filet (6) à profil cylindrique sur **n** tour en fonction de la longueur totale de la vis osseuse (1), et une cinquième partie d'ancrage (11) comportant une âme conique (4**a**) et deux filets (5, 6) à profil conique formant la pointe de ladite vis osseuse.

2. Vis osseuse à double filet discontinu suivant la revendication 1, **caractérisée en ce que** la première partie d'ancrage (7) comprend deux filets (5, 6) à profil cylindrique de pas identique décalés sur la périphérie de l'âme conique (4**a**) de 180° degré.

3. Vis osseuse à double filet discontinu suivant la revendication 1, **caractérisée en ce que** la quatrième partie d'ancrage (10) comprend un filet cylindrique (6) qui est différent du filet cylindrique (5) prévu pour la seconde partie (8).

4. Vis osseuse à double filet discontinu suivant la revendication 1, **caractérisée en ce qu'**elle comporte un alésage interne (13).

5. Vis osseuse à double filet discontinu suivant la revendication 1, **caractérisée en ce que** la tête d'entraînement (2) est constituée d'une tête à profil sphérique (2**a**) présentant dans sa partie interne une empreinte (2**b**) pour la mise en place d'un outil.

6. Vis osseuse à double filet discontinu suivant la revendication 1, **caractérisée en ce que** la tête d'entraînement (2) est constituée d'une tête monobloc (2**c**) présentant un profil en forme de U destiné à recevoir une vis serrage pour l'immobilisation d'un dispositif d'ostéosynthèse.

7. Vis osseuse à double filet discontinu suivant la revendication 1, **caractérisée en ce que** la cinquième partie d'ancrage (11) à profil conique présente des filets (5, 6) auto taraudants.

## Patentansprüche

1. Knochenschraube (1) mit doppeltem diskontinuierlichen Gewinde zum Befestigen einer Prothese oder eines Implantats an einem Knochenabschnitt, umfassend einen Antriebskopf (2), der durch einen Verankerungskörper (3) verlängert wird, der aus einem zylindrisch-konischen Schaft (4) besteht, auf dem sich mindestens zwei zylindrisch-konische Gewinde (5, 6), erstrecken die die Verankerung im Knochen der Schraube (1) sicherstellen, wobei der Verankerungskörper (3) über seine gesamte Länge und ab dem Antriebskopf (2) Verankerungsabschnitte unterschiedlicher Profile umfasst, die eine Verankerung entsprechend den unterschiedlichen Härten und Widerständen des Knochens sicherstellen, **dadurch gekennzeichnet, dass** der Verankerungskörper (3) unterhalb des Antriebskopfes (2) einen ersten Verankerungsabschnitt (7), der aus einem konischen Schaft (4**a**) und zwei Gewinden (5, 6) mit zylindrischem Profil identischer Steigung besteht, einen zweiten Verankerungsabschnitt (8), gebildet aus einem zylindrischen Schaft (4**b**) und einem einzelnen zylindrischen Gewinde (5) auf ¼ der Umdrehung, einen dritten Verankerungsabschnitt (9), versehen mit einem zylindrischen Schaft (4**b**) und zwei Gewinden (5, 6) mit zylindrischem Profil auf ½ Umdrehung, einen vierten Verankerungsabschnitt (10), umfassend einen zylindrischen Schaft (4**b**) und ein einzelnes Gewinde (6) mit zylindrischem Profil auf **n** Umdrehung in Funktion der Gesamtlänge der Knochenschraube (1), und einen fünften Verankerungsabschnitt (11) beinhaltet, beinhaltend einen konischen Schaft (4**a**) und zwei Gewinde (5, 6) mit konischem Profil, die die Spitze der Knochenschraube bilden.

2. Knochenschraube mit doppeltem diskontinuierlichen Gewinde nach Anspruch 1, **dadurch gekennzeichnet, dass** der erste Verankerungsabschnitt (7) zwei Gewinde (5, 6) mit zylindrischem Profil identischer Steigung, auf dem Umfang des konischen Schafts (4**a**) um 180° Grad versetzt, umfasst.

3. Knochenschraube mit doppeltem diskontinuierlichen Gewinde nach Anspruch 1, **dadurch gekennzeichnet, dass** der vierte Verankerungsabschnitt (10) ein zylindrisches Gewinde (6) umfasst, das sich von dem für den zweiten Abschnitt (8) vorgesehenen zylindrischen Gewinde (5) unterscheidet.

4. Knochenschraube mit doppeltem diskontinuierlichen Gewinde nach Anspruch 1, **dadurch gekennzeichnet, dass** sie eine interne Bohrung (13) beinhaltet.

5. Knochenschraube mit doppeltem diskontinuierlichen Gewinde nach Anspruch 1, **dadurch gekennzeichnet, dass** der Antriebskopf (2) aus einem Kopf mit kugelförmigem Profil (2**a**) besteht, der in seinem internen Abschnitt eine Vertiefung (2**b**) zum Ansetzen eines Werkzeugs aufweist.

6. Knochenschraube mit doppeltem diskontinuierlichen Gewinde nach Anspruch 1, **dadurch gekennzeichnet, dass** der Antriebskopf (2) aus einem Monoblock-Kopf (2**c**) besteht, der ein Profil in U-Form aufweist, das dazu bestimmt ist, zur Immobilisierung einer Osteosynthesevorrichtung eine Arretierschraube aufzunehmen.

7. Knochenschraube mit doppeltem diskontinuierlichen Gewinde nach Anspruch 1, **dadurch gekennzeichnet, dass** der fünfte Verankerungsabschnitt (11) mit konischem Profil selbstschneidende Gewinde (5, 6) aufweist.

## Claims

1. Discontinuous double thread bone screw (1) for fixing a prosthesis or an implant against a bone portion, comprising a drive head (2) extending through an anchoring body (3) constituted of a cylindroconical core (4) on which extend at least two cylindroconical threads (5, 6) ensuring the bone anchoring of said screw (1), the anchoring body (3) comprising over the whole of the length thereof and from the drive head (2) of the different profile anchoring portions ensuring an anchoring corresponding to the different hardness and resistances of the bone, **characterised in that** said anchoring body (3) comprises, below the drive head (2), a first anchoring portion (7) constituted of a conical core (4**a**) and two cylindrical profile threads (5, 6) of identical steps, a second anchoring portion (8) formed from a cylindrical core (4**b**) and one single cylindrical thread (5) over ¼ of a rotation, a third anchoring portion (9) provided with a cylindrical core (4**b**) and two cylindrical profile threads (5, 6) over ½ of a rotation, a fourth anchoring portion (10) comprising a cylindrical core (4**b**) and one single cylindrical profile thread (6) over **n** rotations according to the total length of the bone screw (1) and a fifth anchoring portion (11) comprising a conical core (4**a**) and two conical profile threads (5, 6) forming the tip of said bone screw.

2. Discontinuous double thread bone screw according to claim 1, **characterised in that** the first anchoring portion (7) comprises two cylindrical profile threads (5, 6) of identical steps offset over the periphery of the conical core (4**a**) of 180 degrees.

3. Discontinuous double thread bone screw according to claim 1, **characterised in that** the fourth anchoring portion (10) comprises a cylindrical thread (6) which is different from the cylindrical thread (5) provided for the second portion (8).

4. Discontinuous double thread bone screw according to claim 1, **characterised in that** it comprises an inner bore (13).

5. Discontinuous double thread bone screw according to claim 1, **characterised in that** the drive head (2) is constituted of a spherical profile head (2**a**) having in the inner portion thereof, an imprint (2**b**) for the implementation of a tool.

6. Discontinuous double thread bone screw according to claim 1, **characterised in that** the drive head (2) is constituted of a single-piece head (2**c**) having a U-shaped profile intended to receive a clamping screw to immobilise an osteosynthesis device.

7. Discontinuous double thread bone screw according to claim 1, **characterised in that** the fifth cylindrical profile anchoring portion (11) has self-tapping threads (5, 6).
